(19) Europäisches Patentamt
European Patent Office
Office européen des brevets

(11) EP 0 696 575 B1

(12) # EUROPÄISCHE PATENTSCHRIFT

(45) Veröffentlichungstag und Bekanntmachung des Hinweises auf die Patenterteilung:
**22.01.1997 Patentblatt 1997/04**

(51) Int Cl.6: **C07C 213/00**, C07C 215/08, C07D 295/08

(21) Anmeldenummer: **95111813.2**

(22) Anmeldetag: **27.07.1995**

(54) **Verfahren zur Herstellung von optisch aktiven Amino-alkoholen**

Process for the preparation of optically active aminoalcohols

Procédé pour la préparation d'aminoalcools optiquement actifs

(84) Benannte Vertragsstaaten:
**BE CH DE ES FR GB IT LI**

(30) Priorität: **09.08.1994 DE 4428106**

(43) Veröffentlichungstag der Anmeldung:
**14.02.1996 Patentblatt 1996/07**

(73) Patentinhaber: **BAYER AG**
**51368 Leverkusen (DE)**

(72) Erfinder:
- **Antons, Stefan, Dr.**
  **D-51373 Leverkusen (DE)**
- **Beitzke, Bernhard, Dr.**
  **D-51503 Rösrath (DE)**

(56) Entgegenhaltungen:
**DE-A- 4 232 505**

- **JOURNAL OF THE AMERICAN CHEMICAL SOCIETY, Bd. 69, Dezember 1947 DC US, Seiten 3039-3041, HOMER ADKINS ET AL. 'Hydrogenation of Esters to Alcohols over Raney Nickel.'**
- **JOURNAL OF ORGANIC CHEMISTRY, Bd. 24, 1959 EASTON US, Seiten 1847-1854, H. SMITH BROADBENT ET AL. 'Rhenium and its compounds as hydrogenation catalysts.'**
- **JOURNAL OF THE AMERICAN CHEMICAL SOCIETY, Bd. 74, Nr. 4, 21.Februar 1952 DC US, Seite 1096 EDWARD SEGEL 'Hydrogenation of esters of L-alanine and L-leucine over Copper-Chromium oxide catalyst.'**
- **TETRAHEDRON LETTERS, Bd. 33, Nr. 38, 1992 OXFORD GB, Seiten 5517-5518, ATSUSHI ABIKO ET AL. 'An improved, convenient procedure for reduction of amino acids to aminoalcohols..'**
- **REINER LUCKENBACH 'Beilsteins Handbuch der Organischen Chemie, Viertes Ergänzungswerk, Band IV' 1979 , SPRINGER-VERLAG BERLIN . HEIDELBERG . NEW YORK * Seite 1797 - Seite 1798 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 696 575 B1

## Beschreibung

Die vorliegende Erfindung betrifft ein Verfahren zur Herstellung von optisch aktiven Aminoalkoholen durch Reduktion entsprechender Aminosäuren.

Es ist bekannt, optische aktive Aminoalkohole herzustellen, indem man entsprechende Aminosäuren mit $LiAlH_4$ in Ethern reduziert (s. Helv. Chim. Acta 31, 1617 (1948)). Wegen seiner Gefährlichkeit eignet sich $LiAlH_4$ nicht für Anwendungen im technischen Maßstab, sondern allenfalls für Laboransätze.

Das weniger gefährliche $NaBH_4$ reduziert nur Aminosäureester (s. Chem. & Pharm. Bull. 13, 995 (1965)). Das bedeutet zusätzliche Synthesestufen oder besondere Maßnahmen zur Aktivierung von $NaBH_4$ (s. z.B. JACS 78, 2582 (1956)). Die Handhabung von $NaBH_4$ ist immer noch schwierig und deshalb für Arbeiten im technischen Maßstab wenig geeignet.

Es wurde auch schon versucht, Carbonsäuren und -ester katalytisch zu hydrieren (s. Houben-Weyl, Methoden der org. Chemie, 4. Auflage, Band VI/1b, S.103ff). Diese Verfahren erfordern sehr hohe Drucke und Temperaturen. Für die Herstellung optisch aktiver Aminoalkohole sind diese Verfahren nicht geeignet, da bei diesen Reaktionsbedingungen Racemisierungen und Abbaureaktionen stattfinden.

Die katalytische Reduktion mit Rheniumoxid kann man zwar bei niedrigen Temperaturen durchführen, jedoch findet beim Einsatz von Aminosäuren dann nicht nur eine Reduktion der COOH-Gruppe statt, sondern auch eine hydrogenolytische Desaminierung (s. Beispiele 39 und 40 in J. Org. Chem. 24, 1847 (1959)).

Schließlich ist auch die katalytische Reduktion mit Raney-Nickel als Katalysator für die Herstellung von Aminoalkoholen bekannt (s. JACS 69, 3040 (1947) und 70, 3122 (1948)). Man muß dabei allerdings von Aminocarbonsäureestern ausgehen, was zusätzliche Synthesestufen bedeutet, und benötigt große Mengen des Katalysators. Optisch aktive Aminoalkohole sind auf diese Weise bisher nicht hergestellt worden.

Auch in Tetrahedron Letters 33 (38), 5517-5518 (1992) ist eine Reduktion von Aminosäuren zu Aminoalkoholen beschrieben. Als Reduktionsmittel wird $NaBH_4$ x $H_2SO_4$ eingesetzt, aus dem sich in situ hochreaktives und hochtoxisches Diboran ($B_2H_6$), das eigentliche Reduktionsagens, bildet. Dieses Verfahren erfordert deshalb großen sicherheitstechnischen Aufwand, und es ist auf die Reduktion von α-Aminosäuren beschränkt. Die Autoren dieser Literaturstelle haben es sich zur Aufgabe gemacht nur eine "mole-scale"-Synthese und nicht etwa ein in technischen Maßstab durchführbares Verfahren auszuarbeiten.

Es wurde nun ein Verfahren zur Herstellung von optisch aktiven Aminoalkoholen der Formel (II)

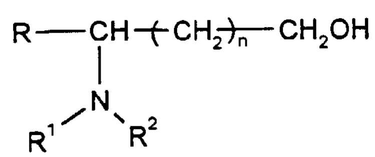

(II),

in der

R für geradkettiges oder verzweigtes $C_1$-$C_{12}$-Alkyl, $C_7$-$C_{12}$-Aralkyl oder $C_6$-$C_{10}$-Aryl,

$R^1$ und $R^2$ unabhängig voneinander für Wasserstoff, geradkettiges oder verzweigtes $C_1$-$C_{12}$-Alkyl oder $C_3$-$C_8$-Cycloalkyl oder

$NR^1R^2$ für einen Rest der Formel

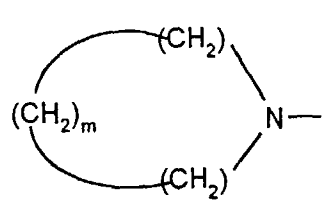

(III)

mit m = einer ganzen Zahl von 2 bis 5 oder

R und $R^1$ gemeinsam für eine

$$-(CH_2)_o-$$

-Gruppierung mit o = einer ganzen Zahl von 2 bis 6 und

n für Null oder eine ganze Zahl von 1 bis 5 stehen,

gefunden, das dadurch gekennzeichnet ist, daß man optisch aktive Aminosäuren der Formel (I)

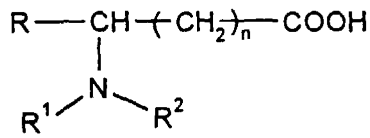

(I),

in der

$R$, $R^1$, $R^2$ und n die bei Formel (II) angegebene Bedeutung haben,

in Gegenwart von Ruthenium-Katalysatoren mit Wasserstoffreduziert, wobei man auf 1 Mol eingesetzte optisch aktive Aminosäure 0,1 bis 10 g elementares Ruthenium oder Rutheniumverbindung oder 1 bis 50g Ruthenium enthaltende Trägerkatalysatoren einsetzt und die Umsetzung in Gegenwart eines Lösungsmittels bei Temperaturen im Bereich 50 bis 150°C und bei Drucken im Bereich 5 bis 300 bar durchführt.

Vorzugsweise setzt man in das erfindungsgemäße Verfahren optisch aktive Aminosäuren der Formel (I) ein, bei denen R für geradkettiges oder verzweigtes $C_1$-$C_4$-Alkyl oder Benzyl, $R^1$ und $R^2$ unabhängig voneinander für Wasserstoff, geradkettiges oder verzweigtes $C_1$-$C_4$-Alkyl oder $NR^1R^2$ für einen Rest der Formel (III) mit m = 3 oder 4 oder R und $R^1$ gemeinsam für

$$-(CH_2)_3- \quad \text{oder} \quad -(CH_2)_4-$$

und n für Null, 1 oder 2 stehen und erhält daraus die entsprechenden optisch aktiven Aminoalkohole.

Als Ruthenium-Katalysatoren kommen elementares Ruthenium und RutheniumVerbindungen in Frage, die beide als solche oder aufgebracht auf einem Trägermaterial zum Einsatz gelangen können. Beispiele für Katalysatoren sind: feinverteiltes elementares Ruthenium, Rutheniumoxide, Rutheniumhydroxide und Rutheniumhalogenide. Als Trägermaterial kommen beispielsweise Kohlen, Aluminiumoxide, Siliciumdioxide, Silikate, Erdalkalicarbonate und Erdalkalisulfate in Frage. Trägerkatalysatoren können beispielsweise 1 bis 20 Gew.-% elementares Ruthenium oder die entsprechende Menge Rutheniumverbindungen enthalten.

Bezogen auf 1 Mol eingesetzte optisch aktive Aminosäure setzt man 0,1 bis 10 g elementares Ruthenium oder Rutheniumverbindungen oder 1 bis 50 g Ruthenium enthaltenden Trägerkatalysatoren ein.

Die erfindungsgemäße Reduktion wird in Gegenwart eines Lösungsmittels für die optisch aktiven Aminosäuren und optisch aktiven Aminoalkohole durchgeführt. Als Lösungsmittel kommen beispielsweise Wasser, mit Wasser mischbare organische Lösungsmittel und Gemische aus beiden in Frage. Als mit Wasser mischbare Lösungsmittel seien niedere Alkohole und mit Wasser mischbare Ether genannt. Bevorzugte Lösungsmittel sind Wasser und Gemische, die Wasser und niedrige Alkohole oder Tetrahydrofuran enthalten.

Bevorzugte Reaktionsbedingungen für die erfindungsgemäße Reduktion sind z.B. 70 bis 130°C und 50 bis 200 bar. Man kann gegebenenfalls auch so verfahren, daß man die Reduktion bei einem relativ niedrigen Druck beginnt, z.B. bei 50 bis 150 bar, und sie bei relativ höheren Drucken zu Ende führt, z.B. bei 150 bis 300 bar. Die Reaktion ist beendet, wenn kein Wasserstoff mehr aufgenommen wird, was im allgemeinen nach 10 bis 50 Stunden der Fall ist.

Zur Aufarbeitung des Reaktionsgemisches kann man beispielsweise zunächst abkühlen, den Katalysator abtrennen, z.B. durch Filtration, die vorhandenen leicht flüchtigen Bestandteile (i.a. Lösungsmittel und Reaktionswasser) durch Destillation, gegebenenfalls unter schwach vermindertem Druck, destillieren und den Rückstand im Vakuum fraktionieren. Den abgetrennten Katalysator kann man wiederverwenden, ebenso das Lösungsmittel.

Das erfindungsgemäße Verfahren kann kontinuierlich oder diskontinuierlich durchgeführt werden.

Die überraschenden Vorteile des erfindungsgemäßen Verfahrens sind, daß damit optisch aktive Aminoalkohole auf einfache Weise, bei relativ niedriger Temperatur, mit wenig Aufwand und in hoher Selektivität (Enantiomerenüberschuß ee meist über 90 %) zugänglich sind.

**Beispiel 1**

In einem 1,3 l Edelstahl-Autoklaven wurden 4 g Ru-Mohr und 89 g L-Alanin in 700 g Wasser vorgelegt. Nach Spülen mit Stickstoff wurde der Autoklav verschlossen und 100 bar Wasserstoff aufgedrückt. Innerhalb von 2 Stunden wurde die Temperatur auf 100°C angehoben und der Wasserstoffdruck auf 200 bar erhöht. Nach 30 Stunden Reaktionszeit wurde auf Raumtemperatur abgekühlt, entspannt, aus dem Reaktionsgemisch der Katalysator durch Filtration abgetrennt und aus dem Filtrat das Wasser abdestilliert. Der erhaltene Rückstand wurde unter Stickstoff bei 10 mbar fraktioniert destilliert. Es wurden 31 g reines L-Alaninol (Kp 74°C), $[\alpha]_D^{20}$ = +16,9, ee = 95 % (hier und in den anderen Beispielen gaschromatographisch bestimmt) erhalten. Als Rückstand hinterblieben 50 g L-Alanin.

**Beispiele 2 bis 7**

Es wurde verfahren wie in Beispiel 1, jedoch wurden andere Katalysatoren eingesetzt. Einzelheiten sind aus der Tabelle 1 ersichtlich.

Tabelle 1

| Beispiel Nr. | Katalysator | | ee des erhaltenen Alaninols (%) |
|---|---|---|---|
| | Art | Menge (g) | |
| 2 | 10 Gew.-% Ru auf Kohle | 20 | 94 |
| 3 | $RuO_2$ | 5 | 95 |
| 4 | 5 Gew.-% Ru auf $Al_2O_3$ | 20 | 98,5 |
| 5 | $RuO_2$ | 5 | 93 |
| 6 | 5 Gew.-% Ru auf Kohle | 20 | 97 |
| 7 | 5 Gew.-% Ru auf Kohle | 20 | 98 |

**Beispiele 8 und 9**

Es wurde verfahren wie bei Beispiel 1, jedoch wurden 5 g $RuO_2$ als Katalysator eingesetzt und andere Temperaturen angewendet.

**Beispiel 8**

80°C, ee des erhaltenen L-Alaninols 98 %.

**Beispiel 9**

110°C, ee des erhaltenen L-Alaninols 93 %.

**Beispiele 10 und 11**

In einer kontinuierlich arbeitenden Apparatur wurden bei 200 bar Wasserstoffdruck pro Stunde 30 g einer 10 gew.-%igen wäßrigen Lösung von L-Alanin über 25 g eines Katalysators geleitet, der 5 Gew.-% Ruthenium auf Kohle enthielt. L-Alaninol wurde bei 100°C in einem ee von 92 % und bei 120°C in einem ee von 89 % erhalten.

**Beispiel 12**

Beispiel 3 wurde fünfmal wiederholt, wobei stets der beim vorhergehenden Ansatz abgetrennte Katalysator wieder eingesetzt wurde. Es wurde keine Änderung am Enantiomerenüberschuß des erhaltenen L-Alaninols festgestellt.

**Beispiele 13 bis 15**

Der nach Beispiel 12 abgetrennte Katalysator wurde nacheinander in Reduktionen analog Beispiel 1 eingegeben, bei denen jedoch entsprechende Mengen andere Lösungsmittel zum Einsatz kamen.

**Beispiel 13**

Gemisch aus 80 Gew.-% Tetrahydrofuran und 20 Gew.-% Wasser, ee des erhaltenen L-Alaninols 95 %.

**Beispiel 14**

Gemisch aus 80 Gew.-% Methanol und 20 Gew.-% Wasser, ee des erhaltenen L-Alaninols 94 %.

**Beispiel 15**

Gemisch aus 80 Gew.-% i-Propanol und 20 Gew.-% Wasser, ee des erhaltenen L-Alaninols 95 %.

**Beispiele 16 bis 18**

Es wurde verfahren wie in Beispiel 1, jedoch wurden entsprechende Mengen anderer Aminosäuren eingesetzt. Einzelheiten sind aus Tabelle 2 ersichtlich.

Tabelle 2

| Beispiel Nr. | eingesetzte Aminosäure | | ee des erhaltenen Aminoalkohols (%) |
|---|---|---|---|
| | Art | $[\alpha]_D^{20}$ | |
| 16 | L-Leucin | + 3,1 | 34 |
| 17 | L-Valin | + 16,6 | 97 |

**Beispiel 18** (zum Vergleich)

Es wurde verfahren wie in Beispiel 1, jedoch als Katalysator 5 g Kupferchromit eingesetzt. Es fand keine Umsetzung statt.

Bei Wiederholungen dieses Beispiels bei höheren Reaktionstemperaturen wurde festgestellt, daß bis 150°C keine Umsetzung stattfand und bei 160°C neben Alaninol weitere Reaktionsprodukte auftraten und das Alaninol weitgehend racemisiert wird.

**Beispiel 19** (zum Vergleich)

Es wurde verfahren wie in Beispiel 1, jedoch wurden als Katalysator 5 g Raney-Nickel eingesetzt.
Es wurde beobachtet, daß sich das Nickel teilweise auflöste. Alaninol konnte nicht isoliert werden.

**Patentansprüche**

**1.** Verfahren zur Herstellung von optisch aktiven Aminoalkoholen der Formel (II)

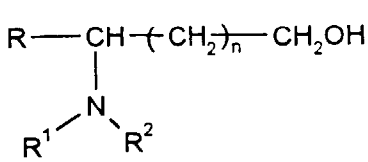

(II),

in der

R für geradkettiges oder verzweigtes $C_1$-$C_{12}$-Alkyl, $C_7$-$C_{12}$-Aralkyl oder $C_6$-$C_{10}$-Aryl,

$R^1$ und $R^2$ unabhängig voneinander für Wasserstoff, geradkettiges oder verzweigtes $C_1$-$C_{12}$-Alkyl oder $C_3$-$C_8$-Cycloalkyl oder

$NR^1R^2$ für einen Rest der Formel

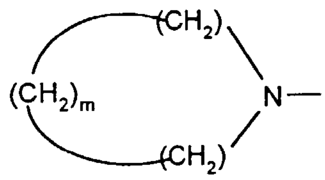

(III)

mit m = einer ganzen Zahl von 2 bis 5 oder

R und $R^1$ gemeinsam für eine

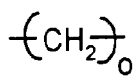

Gruppierung mit o = einer ganzen Zahl von 2 bis 6 und

n für Null oder eine ganze Zahl von 1 bis 5 stehen,

dadurch gekennzeichnet, daß man optisch aktive Aminosäuren der Formel

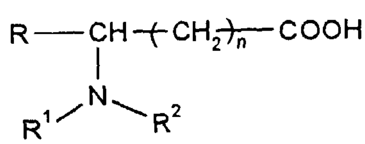

(I),

in der
R, $R^1$, $R^2$ und n die bei Formel (II) angegebene Bedeutung haben,
in Gegenwart von Ruthenium-Katalysatoren mit Wasserstoffreduziert wobei man auf 1 Mol eingesetzte optisch aktive Aminosäure 0,1 bis 10 g elementares Ruthenium oder Rutheniumverbindungen oder 1 bis 50 g Ruthenium enthaltende Trägerkatalysatoren einsetzt und die Umsetzung in Gegenwart eines Lösungsmittels bei Temperaturen im Bereich 50 bis 150°C und bei Drucken im Bereich 5 bis 300 bar durchführt.

**2.** Verfahren nach Anspruch 1, dadurch gekennzeichnet, daß man als Lösungsmittel Wasser, mit Wasser mischbare organische Lösungsmittel oder Gemische aus beiden einsetzt.

**3.** Verfahren nach Ansprüchen 1 bis 2, dadurch gekennzeichnet, daß man das Reaktionsgemisch aufarbeitet, indem man zunächst abkühlt, den Katalysator abtrennt, die vorhandenen leicht flüchtigen Bestandteile abdestilliert und den Rückstand im Vakuum fraktioniert.

**4.** Verfahren nach Anspruch 3, dadurch gekennzeichnet, daß man den abgetrennten Katalysator wiederverwendet.

## Claims

**1.** Process for preparing optically active amino alcohols of the formula (II)

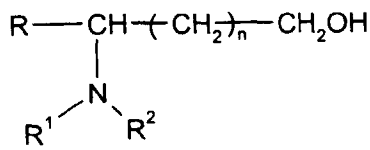

(II),

where

R represents straight-chain or branched $C_1$-$C_{12}$-alkyl, $C_7$-$C_{12}$-aralkyl or $C_6$-$C_{10}$-aryl,

$R^1$ and $R^2$ represent, independently of one another, hydrogen, straight-chain or branched $C_1$-$C_{12}$-alkyl or $C_3$-$C_8$-cycloalkyl or

$NR^1R^2$ represents a radical of the formula

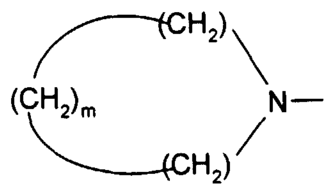

(III)

where m = an integer from 2 to 5 or

R and $R^1$ together represent a

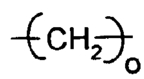

group where o = an integer from 2 to 6 and

n represents zero or an integer from 1 to 5,

characterized in that optically active amino acids of the formula

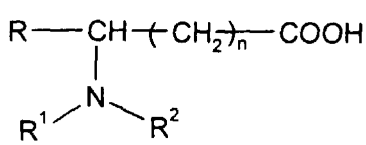

(I),

where
R, $R^1$, $R^2$ and n are as defined for formula (II),
are reduced with hydrogen in the presence of ruthenium catalysts, where from 0.1 to 10 g of elemental ruthenium or ruthenium compounds or from 1 to 50 g of ruthenium-containing supported catalysts are used per 1 mol of optically active amino acid used and the reaction is carried out in the presence of a solvent at temperatures in the range from 50 to 150°C and at pressures in the range from 5 to 300 bar.

**2.** Process according to Claim 1, characterized in that the solvent used is water, water-miscible organic solvents or mixtures of the two.

**3.** Process according to either of claims 1 and 2, characterized in that the reaction mixture is worked up by first cooling, separating off the catalyst, distilling off the readily volatile constituents present and fractionating the residue in vacuo.

**4.** Process according to Claim 3, characterized in that the catalyst separated off is reused.

**Revendications**

**1.** Procédé pour la préparation d'amino-alcools optiquement actifs répondant à la formule (II)

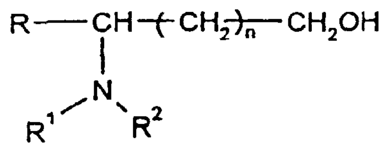

(II),

dans laquelle

R représente un groupe alkyle en $C_1$-$C_{12}$ à chaîne droite ou ramifiée, un groupe aralkyle en $C_7$-$C_{12}$ ou un groupe aryle en $C_6$-$C_{10}$,

$R^1$ et $R^2$ représentent, indépendamment l'un de l'autre, un atome d'hydrogène, un groupe alkyle en $C_1$-$C_{12}$ à chaîne droite ou ramifiée ou un groupe cycloalkyle en $C_3$-$C_8$, ou

$NR^1R^2$ représente un radical de formule

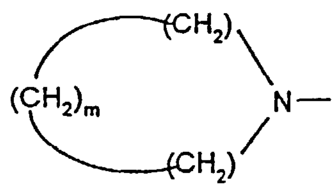

(III)

dans laquelle m = un nombre entier de 2 à 5, ou

R et $R^1$ représentent ensemble un groupement $-(CH_2)-_o$ où o = un nombre entier de 2 à 6, et

n représente zéro ou un nombre entier de 1 à 5,

caractérisé en ce qu'on réduit avec de l'hydrogène des amino-acides optiquement actifs répondant à la formule

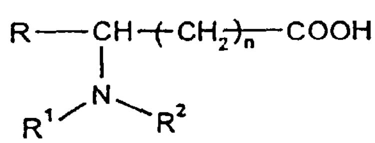

(I),

dans laquelle
R, $R^1$, $R^2$ et n ont la signification indiquée à la formule (II),
en présence de catalyseurs de ruthénium, dans lequel, pour 1 mole d'amino-acide optiquement actif mis en oeuvre, on met en oeuvre de 0,1 à 10 g de ruthénium élémentaire ou de composés de ruthénium, ou encore de 1 à 50 g

de catalyseurs supportés contenant du ruthénium, et on effectue la mise en réaction en présence d'un solvant à des températures dans le domaine de 50 à 150°C et sous des pressions dans le domaine de 5 à 300 bar.

**2.** Procédé selon la revendication 1, caractérisé en ce que, comme solvant, on met en oeuvre de l'eau, des solvants organiques miscibles à l'eau ou encore des mélanges des deux.

**3.** Procédé selon les revendications 1 et 2, caractérisé en ce qu'on traite le mélange réactionnel d'abord en le refroidissant, en séparant le catalyseur, en séparant par distillation les constituants très volatils présents et en fractionnant le résidu sous vide.

**4.** Procédé selon la revendication 3, caractérisé en ce qu'on réutilise le catalyseur séparé.